(19) Europäisches Patentamt
European Patent Office
Office européen des brevets

(11) Veröffentlichungsnummer: **0 051 301**
**A1**

# (12) EUROPÄISCHE PATENTANMELDUNG

(21) Anmeldenummer: **81109378.0**

(22) Anmeldetag: **30.10.81**

(51) Int. Cl.³: **C 07 C 103/52**
C 07 D 209/42, A 61 K 31/02

(30) Priorität: **03.11.80 US 203769**
**03.03.81 US 239918**

(43) Veröffentlichungstag der Anmeldung:
**12.05.82 Patentblatt 82/19**

(84) Benannte Vertragsstaaten:
**AT BE CH DE FR IT LI LU NL SE**

(71) Anmelder: **CIBA-GEIGY AG**
**Patentabteilung Postfach**
**CH-4002 Basel(CH)**

(72) Erfinder: **Renfroe, Harris Burt, Dr.**
**12 Stonehedge Drive**
**West Nyack New York 10994(US)**

(72) Erfinder: **Stanton, James L., Dr.**
**45 Lincoln Avenue**
**Ossining New York 10562(US)**

(72) Erfinder: **Sele, Alex**
**Langmattstrasse 14**
**CH-4132 Muttenz(CH)**

(74) Vertreter: **Zumstein sen., Fritz, Dr. et al,**
**Bräuhausstrasse 4**
**D-8000 München 2(DE)**

## (54) 1-Carboxy-azaalkanoylindolin-2-carbonsäuren, Verfahren zu ihrer Herstellung, pharmazeutische Präparate enthaltend diese Verbindungen, sowie ihre therapeutische Verwendung.

(57) Die Erfindung betrifft hypotensive, antihypertensive und bradykardische Verbindungen der Formel I

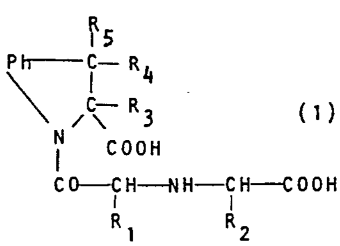

(1),

worin Ph unsubstituiertes 1,2-Phenylen oder durch einen bis drei, gleiche oder verschiedene Substituenten der Gruppe Niederalkyl, Niederalkoxy, Niederalkylendioxy, Hydroxy, Halogen oder Trifluormethyl substituiertes 1,2-Phenylen bedeutet, $R_1$ für Wasserstoff, Niederalkyl oder Amino-niederalkyl steht, $R_2$ Niederalkyl oder HPh-Niederalkyl bedeutet, und jedes der Symbole $R_3$, $R_4$ und $R_5$ für Wasserstoff oder Niederalkyl steht; ihre Amide, Mono- oder Diniederalkylamide, Niederalkylester, (Amino, Mono- oder Diniederalkylamino, Carboxy oder Carboniederalkoxy)-niederalkylester, und ihre Salze. Sie können z.B. durch Hydrolyse einer Verbindung der Formel

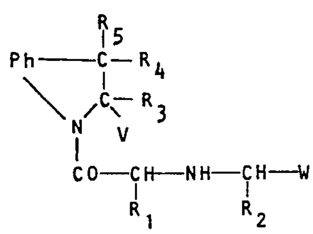

worin mindestens eines der Symbole V und W Cyan bedeutet, und das andere für die genannte freie, amidierte oder veresterte Carboxygruppe steht, hergestellt werden.

EP 0 051 301 A1

CIBA-GEIGY AG
Basel (Schweiz)

4-13120/ 1+2/+

# 1-Carboxy-azaalkanoylindolin-2-carbonsäuren, Verfahren zu ihrer Herstellung, pharmazeutische Präparate enthaltend diese Verbindungen, sowie ihre therapeutische Verwendung.

1-Alkanoylindolin-2-carbonsäuren und ihre 5,6-Dihydroxy-Derivate, nämlich die N-acylierten Cyclodopaderivate, sind in Nippon Kagaku Zasshi 87, 760 (1966), im US-Patent 3,796,723 bzw. In Helv. Chim. Acta 53. 1701 (1970), z.B. als Beispiele für die Synthese von O- und/oder N-Acylverbindungen, beschrieben. Auch 1-(Carboxy-azaalkanoyl oder azaaralkanoyl)-(azetidin, pyrrolidin oder piperidin)-2-carbonsäuren und ihre funktionellen Derivate sind, z.B. aus dem europäischen Patent 0012 401 als Verbindungen mit antihypertensiver Wirkung bekannt.

Es wurde nun überraschend gefunden, dass entweder durch die Einführung einer Carboxygruppe und eines Aza-Teils in die vorher genannten Indoline, oder durch Erweiterung der obigen Pyrrolidine zu Indolin-Ringsystemen, überlegene antihypertensive Mittel erhalten werden.

Die vorliegende Erfindung betrifft daher die neuen 1-Carboxy-(azaalkanoyl oder azaaralkanoyl)-indolin-2-carbonsäuren der allgemeinen Formel I

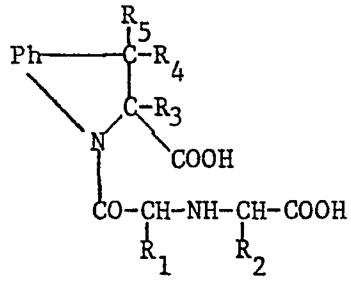

(I),

worin Ph unsubstituiertes 1,2-Phenylen oder durch einen bis drei, gleiche oder verschiedene Substituenten der Gruppe Niederalkyl, Niederalkoxy, Niederalkylendioxy, Hydroxy, Halogen oder Trifluormethyl substituiertes 1,2-Phenylen bedeutet, $R_1$ für Wasserstoff, Niederalkyl oder Amino-niederalkyl steht, $R_2$ Niederalkyl oder HPh-Niederalkyl bedeutet, und jedes der Symbole $R_3$, $R_4$ und $R_5$ für Wasserstoff oder Niederalkyl steht; ihre Amide, Mono- oder Di-niederalkylamide, Niederalkylester, (Amino, Mono- oder Di-niederalkylamino, Carboxy oder Carboniederalkoxy)-niederalkylester, und ihre Salze, insbesondere ihre therapeutisch verwendbaren Salze, sowie Verfahren zu ihrer Herstellung, pharmazeutische Präparate enthaltend diese Verbindungen, sowie ihre therapeutische Verwendung.

Die 1,2-Phenylengruppe Ph und/oder die Phenylgruppe HPh sind vorzugsweise unsubstituiert oder monosubstituiert. Ihre Substituenten können durch die folgenden Gruppen illustriert werden: Niederalkyl, z.B. Methyl, Aethyl, n- oder iso-Propyl oder -Butyl; Niederalkoxy, z.B. Methoxy, Aethoxy, n- oder iso-Propoxy oder -Butoxy; Niederalkylendioxy, z.B. Methylendioxy, 1,1- oder 1,2-Aethylendioxy; Hydroxy; Halogen, z.B. Fluor, Chlor oder Brom; oder Trifluormethyl.

Eine Niederalkylgruppe $R_1$ und/oder $R_2$ ist vorzugsweise Methyl, Aethyl, n- oder iso-Propyl oder -Butyl. Die Amino-niederalkylgruppe $R_1$ bedeutet vorzugsweise $\omega$-Amino-(äthyl, propyl, butyl oder pentyl); und die Aryl-niederalkylgruppe ist auch vorzugsweise HPh-Methyl oder $\omega$-HPh-(Aethyl oder Propyl).

Jedes der Symbole $R_3$, $R_4$ und $R_5$ bedeutet vorzugsweise Wasserstoff, aber auch Niederalkyl, vorzugsweise Methyl, oder eine andere vorher genannte Niederalkylgruppe.

Der Ausdruck "nieder" definiert in den oben oder nachfolgend genannten organischen Resten oder Verbindungen solche mit höchstens 7,

vorzugsweise 4, insbesondere 1 oder 2 Kohlenstoffatomen.

Die genannten funktionellen Derivate, in welchen entweder eine oder die beiden Carboxygruppen verestert oder amidiert sind, sind vorzugsweise Mono- oder Bis-niederalkylester, z.B. Methyl-, Aethyl-, n- oder iso-Propyl- oder -Butylester; Mono- oder Bis-amide, oder die entsprechend N-alkylierten Amide, z.B. Mono- oder Dimethylamid. Die genannten substituierten Niederalkylester sind vorzugsweise Halbester mit einer freien Indolin-2-carboxygruppe, z.B. die ω-(Amino, Mono- oder Dimethylamino, Carboxy oder Carbäthoxy)-(äthyl, propyl oder butyl)-ester.

Salze sind vorzugsweise therapeutisch verwendbare Salze, z.B. Metall- oder Ammoniumsalze der genannten Säuren, insbesondere Alkalimetall- oder Erdalkalimetallsalze, z.B. Natrium-, Kalium-, Magnesium oder Calciumsalze; in erster Linie leicht kristallisierende Ammoniumsalze. Diese werden abgeleitet von Ammoniak oder organischen Aminen, z.B. Mono-, Di- oder Tri-nieder-(alkyl, cycloalkyl oder hydroxyalkyl)-aminen, Niederalkylendiaminen oder (Hydroxy-niederalkyl oder Aryl-niederalkyl)-niederalkylammonium Basen, z.B. Methylamin, Diäthylamin, Triäthylamin, Dicyclohexylamin, Triäthanolamin, Aethylendiamin, Tris-(hydroxymethyl)-aminomethan oder Benzyl-trimethylammoniumhydroxid. Die genannten basischen (Amino, Mono- oder Di-niederalkylamino)-niederalkylester bilden auch Säureadditionssalze. Diese werden vorzugsweise mit solchen Säuren, welche therapeutisch verwendbare Säureadditionssalze ergeben, hergestellt. Säuren, die therapeutisch verwendbare Säureadditionssalze ergeben, sind z.B. anorganische Säuren, wie Halogenwasserstoffsäuren, z.B. Chlorwasserstoff- oder Bromwasserstoffsäure, oder Schwefel-, Phosphor-, Salpeter- oder Perchlorsäure; oder vorzugsweise organische Säuren, wie aliphatische oder aromatische Carbon- oder Sulfonsäuren, z.B. Ameisen-,

Essig-, Propion-, Bernstein-, Glykol-, Milch-, Aepfel-, Wein-, Zitronen-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Aethansulfon-, Hydroxyäthansulfon-, Aethylensulfon-, Halogenbenzolsulfon-, Toluolsulfon-, Naphthalinsulfon-, Sulfanil- oder Cyclohexylsulfaminsäure; oder die Ascorbinsäure.

Die Verbindungen der Erfindung zeigen wertvolle pharmakologische Eigenschaften, in erster Linie hypotensive, anthihypertensive und bradikardische Wirkungen, welche sie unter anderem aufgrund ihrer Hemmwirkung auf das Enzym, welches das Angiotensin umwandelt, hervorrufen. Diese pharmakologischen Eigenschaften können durch in vivo oder in vitro Tierversuchen, vorzugsweise an Säugetieren, wie Ratten, Katzen, Hunden oder ihren isolierten Organen, als Testobjekte nachgewiesen werden. Die Tiere können entweder normotensiv oder hypertensiv, z.B. genetisch hypertensive Ratten, oder renal hypertensive Ratten oder Hunde, und Hunde, denen Natrium entzogen worden ist, sein. Die genannten Verbindungen können ihnen enteral oder parenteral, vorzugsweise oral oder intravenös, z.B. durch Gelatine-Kapseln oder in Form von Stärke enthaltenden Suspensionen bzw. wässerigen Lösungen, verabreicht werden. Die verwendete Dosis kann in einem Bereich von ungefähr zwischen 0,01 und 50 mg/kg/Tag, vorzugsweise ungefähr 0,1 und 25 mg/kg/Tag, insbesondere ungefähr 1 und 10 mg/kg/Tag liegen.

Die in vivo blutdrucksenkende Wirkung wird entweder direkt mit einem Katheter, der z.B. in die femurale Arterie eines Hundes eingeführt ist, oder indirekt durch Sphygmomanometrie am Rattenschwanz, und einem Uebertragungsinstrument registriert. Der Blutdruck wird vor und nach der Verabreichung des Wirkstoffes in mmHg bestimmt. So sind z.B. die repräsentativen Verbindungen dieser Erfindung, welche in den Beispielen illustriert werden, in hypertensiven Ratten oder Hunden

bereits bei einer Verabreichung in niedrigen oralen Dosen von 10 mg/kg/Tag oder darunter, sehr wirksam.

Die neuen Verbindungen zeigen auch eine Hemmwirkung auf das Ansprechen des Blutdrucks von normotensiven Ratten auf Angiotensin I. Das Enzym Renin verursacht unter normalen Verhältnissen eine spezifische Hydrolyse des zirkulierenden Renin-Substrat-Proteins. Diese Hydrolyse ergibt das Angiotensin I, welches durch die Einwirkung des genannten Enzyms, welches das Angiotensin umwandelt, in das stark gefässverengernde Angiotensin II weiter hydrolysiert wird. Durch Hemmung des genannten Enzyms wird die Entstehung des Angiotensins II aus I verhindert. Diese Hemmung dämpft daher jedes Ansprechen des Blutdrucks nach einer Angiotenin-I-Attacke.

Der entsprechende in vivo Test wird mit männlichen, normotensiven Ratten, welche mit 100-120 mg/kg intraperitoneal verabreichtem Natriumsalz des Aethyl-(1-methylpropyl)-malonylthioharnstoffs narkotisiert sind, vorgenommen. Eine femurale Arterie und eine Wadenvene werden mit je einer Hohlnadel für die direkte Messung des Blutdrucks und für die intravenöse Verabreichung des Angiotensins I und der erfindungsgemässen Verbindungen, versehen. Nach der Stabilisierung des basalen Blutdrucks, wird die Druckänderung nach drei, in 5-minutigen Intervallen durchgeführten, Reizungen mit 0,33 µg/kg Angiotensin I i.v., bestimmt. Druckänderungen werden auch 5, 10, 15, 30 und 60 Minuten nach entweder intravenöser oder peroraler Verabreichung (mit Magensonde) der zu prüfenden Verbindungen bestimmt. Die letztgenannten Druckänderungen werden mit den Anfangswerten verglichen. Jede festgestellte Abnahme vom Ansprechen des Blutdrucks ist ein Hinweis auf die Hemmung des Enzyms, welches das Angiotensin umwandelt. Diese Abnahme kann bis zu 80%, nach Dosen von 10 mg/kg i.v. oder 50 mg/kg p.o., betragen und bis 60 Minuten lang andauern.

Die in vitro Hemmung des Enzyms, welches das Angiotensin umwandelt, kann bei den Verbindungen dieser Erfindung analog zu Biochim. Biophys. Acta 293, 451 (1973) nachgewiesen werden. Gemäss dieser Methode werden die genannten Verbindungen in ungefähr 1 mMol Konzentrationen in Phosphatpuffer, welcher von aussen mit Eis gekühlt wird, aufgelöst. Zu diesen Lösungen gibt man zuerst verschiedene µl-Mengen von 1 mMolarem Histidyl-leucin in Phosphatpuffer und dann 100 µl von 5mMolarem Hippuryl-histidyl-leucin in Phosphatpuffer und 50 µl des Angiotensin-umwandelnden Enzyms. Dieses Enzym wird aus Lungen von erwachsenen männlichen Kaninchen in Tris-Puffer, der Kalium- und Magnesiumchlorid und auch Rohrzucker enthält, frisch hergestellt. Die genannten Lösungen werden 30 Minuten bei 37° inkubiert und die weitere Reaktion wird durch Hinzufügen von 0,75 ml einer 0,6-normalen wässerigen Natriumhydroxidlösung gestoppt. Dann gibt man bei Zimmertemperatur 100 µl o-Phthalaldehyd und nach 10 Minuten 100 µl 6-normaler Chlorwasserstoffsäure dazu. Die Proben werden in einem Spektrophotometer bei 360 nm mit Wasser verglichen und ihre optische Dichte wird bestimmt. Diese Werte werden durch einen Konversionsfaktor einer Standard-Kurve angepasst, wobei man die während der genannten Inkubation von 30 Minuten gebildete Histidyl-leucin-Menge in Nanomolen erhält. Die Ergebnisse werden, um den $IC_{50}$-Wert zu bestimmen, gegenüber den Wirkstoff-Konzentrationen graphisch als Kurve dargestellt. Dieser Wert bedeutet diejenige Wirkstoff-Konzentration, welche die Hälfte der Aktivität einer Kontrollprobe, die keinen Wirkstoff enthält, ergibt. Die genannten repräsentativen Verbindungen der vorliegenden Erfindung sind auch in diesem in vitro Testsystem, bis zu dem $IC_{50}$-Wert von 39 nM und darunter, sehr wirksam.

Dementsprechend sind die Verbindungen der Erfindung wertvolle antihypertensive Mittel, insbesondere für die Herabsetzung von hohem Blutdruck (ohne Rücksicht auf die Aetiologie) und/oder bei Herzerkrankungen wie Herzinsuffizienz, und/oder anderen Zuständen, die mit Oedemen oder Ascites einhergehen, z.B. Leberzirrhose. Sie können

auch als Zwischenprodukte zur Herstellung von anderen wertvollen Produkten, insbesondere von pharmakologisch wirksamen Präparaten eingesetzt werden.

Bevorzugte Verbindungen sind diejenigen der Formel I, worin Ph unsubstituiertes 1,2-Phenylen oder durch einen oder zwei, gleiche oder verschiedene Substituenten der Gruppe Niederalkyl, Niederalkoxy, Hydroxy und Halogen, oder durch eine Alkylendioxy- oder Trifluormethylgruppe substituiertes 1,2-Phenylen bedeutet; $R_1$ für Wasserstoff, Niederalkyl oder ω-Aminoniederalkyl steht, $R_2$ Niederalkyl oder ω-HPh-Niederalkyl bedeutet, und jedes der Symbole $R_3$, $R_4$ und $R_5$ für Wasserstoff oder Niederalkyl steht, ihre Amide, Mono- oder Di-niederalkylamide, Niederalkylester, (Amino, Mono- oder Di-niederalkylamino, Carboxy oder Carbo-niederalkoxy)-niederalkylester und Salze, insbesondere therapeutisch verwendbare Alkalimetall-, Erdalkalimetall-, Ammoniumsalze oder Säureadditionssalze.

Bevorzugt sind weiter Verbindungen der Formel I, worin Ph ein gegebenenfalls durch Niederalkyl, Niederalkoxy, Niederalkylendioxy, Hydroxy, Halogen oder Trifluormethyl monosubstituiertes 1,2-Phenylen bedeutet, $R_1$ für Niederalkyl oder ω-Aminoniederalkyl steht, $R_2$ Niederalkyl oder ω-HPh-Niederalkyl bedeutet,und jedes der Symbole $R_3$, $R_4$ und $R_5$ Wasserstoff oder Methyl bedeutet, ihre Amide, Mono- oder Di-niederalkylamide, Niederalkylester, (Amino, Mono- oder Di-niederalkylamino, Carboxy, oder Carbo-niederalkoxy)-niederalkylester und Salze, insbesondere therapeutisch verwendbare Alkalimetall-, Erdalkalimetall-, Ammonium- oder Säureadditionssalze.

Besonders hervorzuheben sind Verbindungen der allgemeinen Formel II

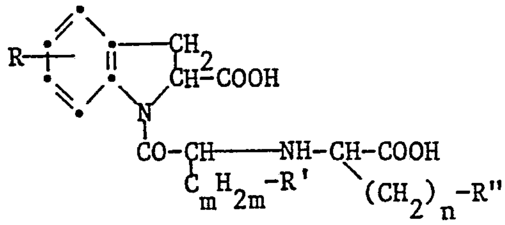

(II),

insbesondere ihre Indolin-2S-chiralen Epimere, worin R Wasserstoff, Alkyl oder Alkoxy mit höchstens je 4 Kohlenstoffatomen, Hydroxy, Halogen oder Trifluormethyl bedeutet, m für eine ganze Zahl von 1 bis 4 steht, n eine ganze Zahl von 1 bis 3 bedeutet, R' Für Wasserstoff oder Amino steht, und R" Wasserstoff oder R-Phenyl bedeutet; ihre Mono- oder Bisamide, die Mono- oder Bis-(niederalkyl- oder ω-amino-niederalkyl)-ester, und Salze, insbesondere therapeutisch verwendbare Alkalimetall-, Ammonium- oder Säureadditionssalze.

Besonders bevorzugt sind Verbindungen der Formel II, worin R Wasserstoff, Methyl, Methoxy, Hydroxy oder Chlor, vorzugsweise in 5-Stellung bedeutet, m für 1 steht, n die Zahl 2 ist, R' für Wasserstoff steht und R" Phenyl bedeutet; ihre Mono- oder Bis-amide, die Mono- oder Bis-(niederalkyl oder ω-amino-niederalkyl)-ester, und Salze, insbesondere therapeutisch verwendbare Alkalimetall-, Ammoniumsalze oder Säureadditionssalze.

Die Verbindungen der vorliegenden Erfindung werden nach an sich bekannten Methoden, vorzugsweise dadurch hergestellt, dass man

1) eine Schiff'sche Base der allgemeinen Formel III

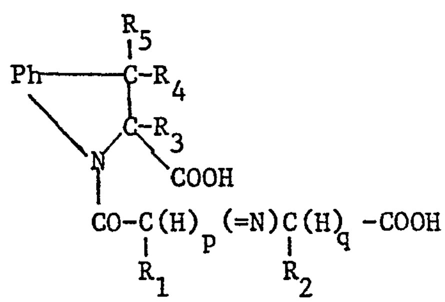

(III)

oder ihre genannten funktionellen Derivate, worin eines der Symbole

p und q für Null steht und das andere 1 bedeutet, hydriert, oder

2) eine Verbindung der allgemeinen Formel IV

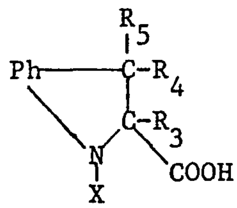

(IV)

oder ihre genannten funktionellen Derivate , mit einem reaktionsfähigen Derivat einer Verbindung der allgemeinen Formel V

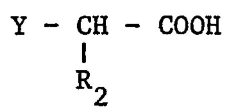

(V),

in welcher X Wasserstoff bedeutet und Y für die Gruppierung HOCO-$CHR_1$-N-$COOR_o$ steht, worin $R_o$ t-Butyl oder Benzyl bedeutet; oder X für CO-$CHR_1$-Z steht und eines der Symbole Y und Z Amino bedeutet und das andere für eine reaktionsfähige veresterte Hydroxygruppe steht, kondensiert, und die genannte $COOR_o$-Gruppe in Wasserstoff umwandelt, oder

3) eine Verbindung der allgemeinen Formel VI

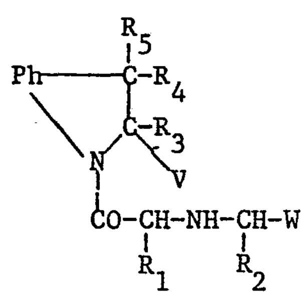

(VI),

worin mindestens eines der Symbole V und W Cyan bedeutet, und das andere für die genannte freie, amidierte oder veresterte Carboxygruppe steht, hydrolysiert oder alkoholysiert, oder

4) in einer Verbindung der allgemeinen Formel VII

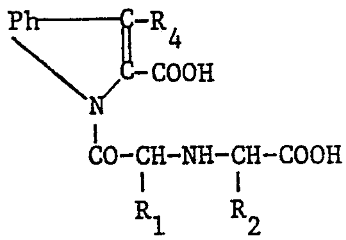

(VII)

oder in ihren genannten funktionellen Derivaten, den Indolteil zum Indolinteil hydriert, und, wenn erwünscht, eine erhaltene Verbindung in eine andere Verbindung der Erfindung umwandelt.

Die Hydrierung von Verbindungen der Formel III wird nach an sich bekannten Methoden, z.B. entweder mit katalytisch aktiviertem oder nascierendem Wasserstoff, wie Wasserstoff in Gegenwart von Platin-, Palladium- oder Nickel-Katalysatoren, oder mit elektrolytisch erzeugtem Wasserstoff, oder durch Einwirkung von Metallen auf Säuren oder Alkohole, durchgeführt. Auch Reduktionsmittel, z.B. einfache oder komplexe Leichtmetallhydride, wie Borane, oder vorzugsweise Alkalimetallhydride oder -cyanborhydride, können verwendet werden.

Reaktionsfähige funktionelle Derivate von Verbindungen der Formel V sind vorzugsweise Ester-halogenide, einfache oder gemischte Anhydride, z.B. die Niederalkyl-halbester der genannten Säurechloride, das cyclische Anhydrid, oder gemischte Essigsäure- oder Cyanessigsäureanhydride. Eine reaktionsfähige veresterte Hydroxygruppe Y oder Z ist vorzugsweise Halogen, z.B. Chlor, Brom oder Jod, oder aromatisches Sulfonyloxy, z.B. Tosyloxy oder Brosyloxy. Die genannte Kondensation von Verbindungen III und IV verläuft entweder spontan oder in Gegenwart von Kondensationsmitteln, z.B. organischen oder anorganischen Basen, wie von den genannten salzbildenden Aminen oder Alkalimetallcarbonaten oder disubstituierten Carbodiimiden. In einer erhaltenen Verbindung, welche die Aza-Schutzgruppe $COOR_o$ enthält, kann die letztere gemäss den aus der Peptidchemie wohlbekannten Verfahren, vorzugsweise durch die vorher beschriebene katalytische Hydrierung, oder durch

die weiter unten geschilderte Hydrolyse, abgespalten werden. Die genannten Ausgangsstoffe sind auch bekannt, oder wenn neu, können sie gemäss einer dem Verfahren 2) analogen Kondensation hergestellt werden.

Die Hydrolyse der Nitrile VI zu den entsprechenden Säuren oder Amiden wird in an sich bekannter Weise, vorzugsweise mit anorganischen Säuren, z.B. Chlorwasserstoff- oder Schwefelsäure, durchgeführt. Die genannte Alkoholyse wird analog, in Gegenwart von den genannten Säuren und den entsprechenden unsubstituierten oder substituierten Niederalkanolen, vorgenommen.

Schliesslich wird die genannte Hydrierung von Indolen VII zu den Indolinen I auch gemäss den konventionellen Hydrierungen von 1-Acylindolen, z.B. mit katalytisch aktiviertem oder nascierenden Wasserstoff, wie Wasserstoff in Gegenwart von Platin-, Palladium-, Rhodium- oder Nickel-Katalysatoren, oder mit elektrolytisch erzeugtem Wasserstoff, oder durch Einwirkung von Metallen auf Säuren oder Alkohole, durchgeführt. Auch Reduktionsmittel, z.B. einfache oder komplexe Leichtmetallhydride, wie Borane, oder vorzugsweise Alkalimetallborhydride oder -cyanborhydride, können verwendet werden. Bevorzugt ist die asymmetrische Hydrierung zu den Indolin-2S-carbonsäuren oder ihren genannten Derivaten. Man verwendet dabei chirale Katalysatoren, z.B. solche, die man aus einem Rhodiumsalz mit (R)-1,2-Bis-(diphenylphosphino)-propan oder (R)-1,2-Bis-(o-anisylphenylphosphino)-äthan und 1,5-Cyclooctadien herstellt.

Funktionelle Derivate von Carbonsäuren, z.B. denjenigen der Formeln III, IV, V, VII, $R_2CHNH_2COOH$ oder $R_2COCOOH$ sind vorzugsweise Ester, z.B. Niederalkylester, wie Aethyl-, t-Butyl- oder Aralkylester, z.B. Benzylester.

Unter einem reaktionsfähigen Derivat einer Carbonsäure sind in dieser Erfindung vorzugsweise Säurehalogenide, reaktionsfähige Ester, einfache oder gemischte Anhydride oder ähnliche aus dem Stand der Technik bekannte Verbindungen, wie diejenigen, welche für die Formel V beschrieben sind, zu verstehen.

Die Ausgangsstoffe sind bekannt oder können, falls sie neu sind, nach an sich bekannten Methoden, z.B. analog wie in den Beispielen beschrieben, hergestellt werden. Neue Ausgangsstoffe bilden ebenfalls einen Gegenstand der Erfindung.

Ausgangsstoffe der Formel III werden vorzugsweise in situ, durch Kondensation einer Verbindung der Formel IV, worin X die Gruppe $R_1COCO$ bedeutet, mit einer Verbindung $R_2CHNH_2COOH$ oder einem ihrer funktionellen Derivate, hergestellt. Sie können auch durch Kondensation einer Verbindung IV, worin X die Gruppe $R_1CHNH_2CO$ bedeutet, mit einer Verbindung der Formel $R_2COCOOH$ oder einem ihrer funktionellen Derivate erhalten werden. Die Kondensationen werden in einem inerten Lösungsmittel in Abwesenheit oder in Gegenwart z.B. einer wasserfreien Mineralsäure oder einer organischen Säure (z.B. Bortrifluorid, Chlorwasserstoff oder Essigsäure),eines anorganischen Salzes (z.B. Magnesiumsulfat) oder eines Molekularsiebes, vorgenommen.

Die Zwischenprodukte der Formel IV, worin X die Gruppe $R_1COCO$ bedeutet, sind bekannt oder können durch Kondensation einer Verbindung der Formel IV oder eines Esters davon, worin X Wasserstoff bedeutet, mit einer gegebenenfalls geschützten (z.B. Dithioketal) Verbindung der Formel $R_1COCOOH$ oder einem ihrer reaktionsfähigen funktionellen Derivate, erhalten werden.

Zwischenprodukte der Formel IV, worin X $R_1CHNH_2CO$ bedeutet, werden durch Kondensation einer Verbindung der Formel IV oder eines ihrer Ester, worin X Wasserstoff bedeutet, mit einer gegebenenfalls N-geschützten (z.B. N-Benzyloxycarbonyl oder t-Butyloxycarbonyl Derivat) Verbindung der Formel $R_1CHNH_2COOH$ oder einem ihrer reaktionsfähigen

funktionellen Derivate, hergestellt werden. Falls nötig, kann die geschützte Gruppe nachher in an sich bekannter Weise oder wie hier unten beschrieben, wieder freigesetzt werden. Die oben oder nachfolgend genannten Kondensationen mit reaktionsfähigen funktionellen Derivaten von Carbonsäuren verlaufen spontan oder in Gegenwart von Kondensationsmitteln, z.B. anorganischen oder organischen Basen, wie Kaliumcarbonat, Pyridin oder Triäthylamin. Die Kondensationen von Carbonsäuren per se können in Gegenwart von Verknüpfungsmitteln, z.B. Dicyclohexyl-carbodiimid oder 1-(3-Dimethylaminopropyl)-3-äthylcarbodiimid, durchgeführt werden.

Verbindungen der Formel IV, worin X die Gruppe $COCHR_1Z$ bedeutet, können durch Acylierung einer Verbindung der Formel IV, worin X Wasserstoff ist, mit einem funktionellen Derivat von $HOCOCHR_1Z$, worin Z eine reaktionsfähige veresterte Hydroxygruppe (z.B. Chlor oder Tosyloxy) oder eine geschützte Aminogruppe , z.B. t-Butyloxycarbonylamino (t-BOC-Amino) oder Carbobenzyloxyamino (Cbo-Amino) bedeutet, und Ueberführung in die Verbindung der Formel IV, worin X die Gruppe $COCHR_1NH_2$ bedeutet, nach an sich bekannten und hier weiter unten beschriebenen Methoden, z.B. Hydrolyse unter sauren Bedingungen oder katalytische Hydrierung, hergestellt werden.

Verbindungen der Formel V, worin Y $HOCO-CHR_1-N-COOR_o$ bedeutet, können vorzugsweise wie folgt hergestellt werden:

a) Umsetzung eines Esters (z.B. eines Niederalkylesters, wie t-Butylester) einer Verbindung der Formel $R_1CHNH_2COOH$ mit einem Ester (z.B. Niederalkylester, wie Aethylester) einer Verbindung der Formel $R_2CO-COOH$, in Gegenwart eines Dehydratisierungsmittels, z.B. Bortrifluorid.

b) Reduktion der erhaltenen Schiff'schen Base nach an sich bekannten Methoden, z.B. durch Hydrierung in Gegenwart von Palladium- oder Nickelkatalysatoren oder mit einem Reduktionsmittel, z.N. Natriumcyanborhydrid.

c) Schutz des Stickstoffs im erhaltenen Azadiester mit einem bekannten reaktionsfähigen funktionellen Derivat von $R_oCOOH$ und

d) Hydrolyse eines erhaltenen N-geschützten Diesters einer Verbindung der Formel V, worin Y $HOCO-CHR_1-N-COOR_o$ bedeutet.

Ausgangsstoffe der Formel VI, worin W Cyan bedeutet, können vorzugsweise ausgehend von einer Verbindung der Formel IV, worin X $COCHR_1NH_2$ bedeutet,und einer Verbindung der Formel $R_2CHY-CN$, worin Y eine reaktionsfähige veresterte Hydroxygruppe bedeutet, hergestellt werden.

Ausgangsstoffe der Formel VII können gemäss dem Verfahren 2), wie vorher beschrieben, erhalten werden. In diesem Verfahren wird eine Verbindung der Formel IV durch die entsprechende Indolverbindung ersetzt, in welcher anstelle von $R_3$ und $R_4$ eine einfache Bindung, welche die die Reste $R_3$ und $R_4$ tragenden Kohlenstoffatome verbindet, vorhanden ist.

Die erhaltenen Verbindungen der Erfindung können in an sich bekannter Weise in andere Verbindungen der Erfindung übergeführt werden. So können z.B. erhaltene Amide oder Ester weiter hydrolysiert oder alkoholisiert (umestert) werden. Dies kann gemäss dem Verfahren 3), oder mit wässerigen Alkalien, z.B. mit Alkalimetall-carbonaten bzw. -hydroxiden, vorgenommen werden. Erhaltene freie Säuren können in an sich bekannter Weise, mit den genannten unsubstituierten oder substituierten Niederalkanolen oder Diazoalkanen verestert, oder in die genannten Metall-, Ammonium- oder in die Säureadditionssalze umgewandelt werden.

So kann z.B. eine erhaltene freie (amphotere) Verbindung in ein entsprechendes Metall, Ammonium- bzw. Säureadditionssalz durch Umsetzung mit einer äquivalenten Menge einer entsprechenden Base, eines basischen Salzes, einer Säure oder eines Ionenaustauschers, umgewandelt werden. Die genannten Säuren werden dabei mit Alkalimetall- oder

Ammoniumhydroxiden oder -carbonaten, und die genannten Säuren, Amide oder Ester mit den oben genannten anorganischen bzw. organischen Säuren behandelt. Ein erhaltenes Salz kann auch in die freie Verbindung, durch Behandlung mit stärkeren Säuren bzw. Basen, vorzugsweise bei einem pH-Wert zwischen ungefähr 3 und 5, übergeführt werden.

Infolge der engen Beziehung zwischen den neuen Verbindungen in freier Form und in Form ihrer Salze sind im vorausgegangenen und nachfolgend unter freien Verbindungen (oder Zwischenprodukten) und Salzen sinn- und zweckgemäss gegebenenfalls auch die entsprechenden Salze bzw. freien Verbindungen zu verstehen.

Erhaltene geometrische oder optische Isomerengemische von obigen Verbindungen der Formeln I bis VII können nach an sich bekannten Methoden, z.B. durch fraktionierte Destillation, Kristallisation und/oder Chromatographie, in die einzelnen Isomeren getrennt werden. Racemische Produkte können in die optischen Antipoden, z.B. durch Trennung ihrer diastereomeren Salze, wie gemäss J. Org. Chem. 43, 3803 (1978), z.B. durch fraktionierte Kristallisation von d- und ℓ-(Tartraten, Mandelaten, Camphersulfonaten oder 1-Naphthyl-1-äthylisocyanaten), oder von d- und ℓ-(α-Methyl-benzylammonium, Cinchonidin, Cinchonin, Chinin, Chinidin, Ephedrin, Dehydroabietylamin, Brucin oder Strychnin)-salzen, gespalten werden. Der bevorzugte Ausgangsstoff der Formel III ist sein 2S-optisches Isomere (Epimere).

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, alkalischen oder sauren Kondensations- oder anderen oben genannten Mitteln, z.B. den üblichen, für den Schutz der Aminogruppe in der Peptidsynthese verwendeten Mitteln, und/oder in einer inerten Atmosphäre, unter Kühlung, bei Zimmertemperatur oder

bei erhöhten Temperaturen, vorzugsweise beim Siedepunkt des verwendeten Lösungsmittels, bei normalem oder erhöhtem Druck, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird.

Im Verfahren der vorliegenden Erfindung werden vorteilhafterweise solche Ausgangsstoffe verwendet, welche zu den im vorstehenden als besonders wertvoll beschriebenen Verbindungen, insbesondere solchen der Formel II, führen und die folgenden chiralen Isomeren sind:

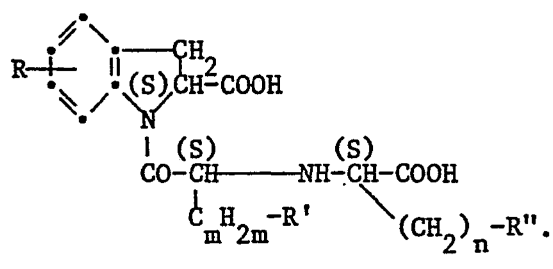

(IIa)

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten

ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, Enzyme der Bindemittel und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1% bis etwa 75%, insbesondere von etwa 1% bis etwa 50%, des Aktivstoffes. Einzeldosen für Säuger mit einem Gewicht von ungefähr 50-70 kg können zwischen ungefähr 5-100 mg des aktiven Bestandteils enthalten.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 15 und 100 mm/Hg, durchgeführt.

Beispiel 1: Ein Gemisch von 1,0 g 1-(S-Alanyl)-indolin-2S-carbonsäure, 3,8 g 4-Phenyl-2-oxobutansäure und 30 ml Wasser wird bei Zimmertemperatur mit wässeriger Natriumhydroxidlösung auf den pH-Wert 7,5 eingestellt. Das Gemisch wird mit 0,8 g Natrium-cyanborhydrid versetzt und über Nacht bei Zimmertemperatur gerührt. Das Gemisch wird dann mit 5 ml konzentrierter Chlorwasserstoffsäure gelöscht und durch eine kurze Säule eines stark sauren Ionenaustauscherharzes (Dowex 50 W - X4) filtriert. Die Säule wird zuerst mit 50%igem wässerigem Aethanol (Beseitigung des Ausgangsstoffes) und dann mit 4%igem (Volumen) wässerigem Ammoniak eluiert. Das letztere Eluat wird eingedampft, der Rückstand in 6 ml Wasser aufgeschlämmt, dessen pH-Wert mit 1-normaler Chlorwasserstoffsäure auf 3 eingestellt und der erhaltene Niederschlag gesammelt. Man erhält die 1-[N-(1-Carboxy-3-phenylpropyl)-S-alanyl]-indolin-2S-carbonsäure der Formel II, worin R=R'=H, m = 1, n = 2 und R" = $C_6H_5$. F. 140-146°, $[\alpha]_D$ = -96° (c = 0,8 in Aethanol).

Der Ausgangsstoff wird wie folgt hergestellt: Man löst 120 g 1-Acetyl-indolin-2-carbonsäure (Nippon Kagaku Zasshi 87, 760 (1966)] und 172 g ℓ-Cinchonidin in 1200 ml heissem Aethanol. Die Lösung wird bei Zimmertemperatur über Nacht, und dann 4 Tage bei 0° stehen gelassen. Das weisse kristalline Salz wird abfiltriert und beseitigt. Das Filtrat wird eingedampft, mit 1000 ml Wasser versetzt und die Lösung mit konzentrierter Chlorwasserstoffsäure auf den pH-Wert 1 eingestellt. Nach 15 Minuten wird das Produkt abfiltriert, dreimal mit 250 ml 2-normaler Chlorwasserstoffsäure, zweimal mit 500 ml Wasser und zweimal mit 100 ml Aethanol gewaschen. Man erhält die 1-Acetyl-indolin-2S-carbonsäure, welche bei 214-215° schmilzt. $[\alpha]_D$ = -133,3° (c = 1,165 in Aethanol).

Eine Suspension von 37,5 g der letztgenannten Verbindung in 380 ml 2-normaler wässeriger Chlorwasserstoffsäure wird zuerst von Sauerstoff durch Durchleiten von Stickstoff (5 Minuten) befreit und dann

2 Stunden unter Rückfluss gekocht. Das Reaktionsgemisch wird auf Zimmertemperatur gekühlt, durch Infusorienerde filtriert, das Filtrat eingedampft und aus Aether-Isopropanol umkristallisiert. Man erhält das Indolin-2S-carbonsäure-hydrochlorid, welches unter Zersetzung bei 133° schmilzt. $[\alpha]_D$ = -70,4° (c = 1 in Aethanol).

Eine Lösung von 34 g der letztgenannten Verbindung wird ohne Kühlung mit trockenem Chlorwasserstoff gesättigt. Das Gemisch wird 2 Stunden bei Zimmertemperatur gerührt und das Lösungsmittel bis zum Anfang der Kristallisation abgedampft. Das Konzentrat wird in 400 ml Diäthyläther gegossen, eine Stunde auf 0° gekühlt und filtriert. Man erhält das Indolin-2S-carbonsäure-äthylester-hydrochlorid, welches bei 179-181° schmilzt. $[\alpha]_D$ = -63° (c = 1,385 in Aethanol).

Ein Gemisch von 18,0 g der letztgenannten Verbindung, 18,5 g N-Carbobenzyloxy-S-alanin, 11 ml Triäthylamin und 300 ml Methylenchlorid wird unter Rühren bei 0° mit 16,7 g 1-(3-Dimethylamino-propyl)-3-äthyl-carbodiimid-hydrochlorid versetzt. Nach einer Stunde lässt man die Temperatur auf Zimmertemperatur steigen und setzt das Rühren über Nacht fort. Dann wird das Gemisch mit Wasser, 2-normaler Chlorwasserstoffsäure und gesättigter wässeriger Natriumhydrogencarbonatlösung gewaschen, getrocknet und eingedampft. Man erhält den 1-(N-Carbobenzyloxy-S-alanyl)-indolin-2S-carbonsäureäthylester.

Ein Gemisch von 30,8 g der letztgenannten Verbindung, 3,6 g Lithiumhydroxid, 100 ml Dimethylformamid und 100 ml Wasser wird 2 Stunden bei Zimmertemperatur gerührt, mit 200 ml konzentrierter Chlorwasserstoffsäure angesäuert und dreimal mit je 100 ml Methylenchlorid extrahiert. Die vereinigten Extrakte werden mit Wasser gewaschen, getrocknet und auf 50 ml konzentriert. Das Konzentrat wird mit 300 ml Diäthyläther verdünnt, dreimal mit je 100 ml gesättigter wässeriger Natriumhydrogencarbonatlösung extrahiert, die kombinierten Extrakte mit

konzentrierter Chlorwasserstoffsäure angesäuert und mit Methylenchlorid extrahiert. Der Extrakt wird getrocknet und eingedampft. Man erhält die 1-(N-Carbobenzyloxy-S-alanyl)-indolin-2S-carbonsäure, welche bei 144-146° schmilzt.

Ein Gemisch von 15 g der letztgenannten Verbindung, 1,0 g Palladiumauf-Kohle Katalysator und 150 ml 90%igem wässerigem Aethanol wird bei Zimmertemperatur und atmosphärischen Druck 2 Stunden hydriert, filtriert und eingedampft. Man erhält die 1-(S-Alanyl)-indolin-2S-carbonsäure, welche bei 205-207° schmilzt. $[\alpha]_D$ = -141,8° (c = 1,1 in Wasser).

Beispiel 2: Ein Gemisch von 0,50 g 1-(S-Alanyl)-indolin-2S-carbonsäure, 0,66 g 4-Phenyl-2-oxobutansäure-äthylester, 0,43 g Triäthylamin, 0,7 g Raney-Nickel, 2 g 3A-Molekularsieb und 20 ml Aethanol wird bei Zimmertemperatur und 2,7 Atmosphären, 18 Stunden hydriert. Das Gemisch wird filtriert, das Filtrat eingedampft und der Rückstand mit 15 ml Essigsäureäthylester und 30 ml Wasser geschüttelt. Das Gemisch wird mit 2-normaler wässeriger Natriumhydroxidlösung auf den pH-Wert 8,6 eingestellt, die wässerige Phase abgetrennt und mit 10 ml Essigsäureäthylester gewaschen. Die wässerige Phase wird mit 4-normaler Chlorwasserstoffsäure auf den pH-Wert 4,25 eingestellt und dreimal mit je 10 ml Essigsäureäthylester extrahiert. Die vereinigten Extrakte werden getrocknet und eingedampft. Man erhält die 1-[N-(1-Carboäthoxy-3-phenylpropyl)-S-alanyl]-indolin-2S-carbonsäure, welche IR-Banden bei 3210, 1745 und 1720 $cm^{-1}$ aufweist. $[\alpha]_D$ = -103,4° (c = 0,6 in Aethanol).

Man löst 0,6 g der letztgenannten Verbindung in 5 ml Diäthyläther und leitet in die Lösung wasserfreien Chlorwasserstoff ein. Der erhaltene Niederschlag wird abfiltriert, mit Diäthyläther gewaschen und getrocknet. Man erhält das entsprechende Hydrochlorid, welches

bei 176-180° schmilzt.

Beispiel 3: Analog den hier beschriebenen Methoden werden die folgenden 1-(Carboxy-azaaralkanoyl)-indolin-2S-carbonsäuren der Formel II hergestellt:

| No. | R | m | R' | n | R" | IR ($cm^{-1}$) oder NMR (ppm) Peaks |
|---|---|---|---|---|---|---|
| 1 | H | 0 | H | 2 | $C_6H_5$ | 3225, 1740, 1715 |
| 2 | H | 1 | H | 1 | $C_6H_5$ | 1.35, 2.85, 4.80 |
| 3 | H | 3 (iso) | H | 2 | $C_6H_5$ | 1.15, 4.85, 7.9-7.1 |
| 4 | H | 4 (n) | $NH_2$ | 2 | $C_6H_5$ | 2.95, 4.95, 7.95-7.1 |
| 5 | 5-$CH_3$ | 1 | H | 2 | $C_6H_5$ | 2.28, 4.72, 6.48 |
| 6 | 5-$OCH_3$ | 1 | H | 2 | $C_6H_5$ | 3.72, 4.80 |
| 7 | 5-OH | 1 | H | 2 | $C_6H_5$ | 3265, 2975, 1735 |
| 8 | 5-Cl | 1 | H | 2 | $C_6H_5$ | 4.90, 7.20, 8.21 |
| 9 | H | 1 | H | 2 | p-$C_6H_4$-Cl | 3195, 1740, 1655 |

Beispiel 4: Ein Gemisch von 5,0 g 1-(S-Alanyl)-indolin-2S-carbonsäure, 22,0 g 2-Oxo-4-phenylbutansäure-äthylester, 6,0 ml Triäthylamin und 33,0 g 3A-Molekularsieb in 75 ml Aethanol wird bei Zimmertemperatur 30 Minuten gerührt. Eine Lösung von 1,34 g Natrium-cyanborhydrid in 25 ml Aethanol wird innerhalb 3 Stunden zugesetzt. Das Reaktionsgemisch wird über Nacht bei Zimmertemperatur gerührt, filtriert und das Filtrat zur Trockene eingedampft. Der Rückstand wird mit 30 ml Methylenchlorid und 50 ml Wasser geschüttelt, wobei man den pH-Wert mit 2-normaler wässeriger Natriumhydroxidlösung auf 9 einstellt. Die wässerige Phase wird abgetrennt und mit 30 ml Methylenchlorid gewaschen. Die wässerige Lösung wird mit konzentrierter Chlorwasserstoffsäure auf den pH-Wert 1 eingestellt, 30 Minuten gerührt, durch Zugabe von 2-normaler Natriumhydroxidlösung auf den pH-Wert 3 eingestellt und dann dreimal mit 50 ml Methylenchlorid extrahiert.

Der Methylenchlorid-Extrakt wird über Magnesiumsulfat getrocknet und zur Trockene eingedampft. Man erhält ein Produkt, das hauptsächlich aus dem gewünschten Diastereoisomeren besteht. Dieses wird auf einer Silicagel-Säule durch Hochdruck-Flüssigkeitschromatographie gereinigt. Als Eluent wird ein 85:15-Gemisch von Essigsäureäthylester und Methanol verwendet. Man erhält die 1-[N-(1S-Carboäthoxy-3-phenyl-propyl)-S-alanyl]-indolin-2S-carbonsäure. Dieses Produkt wird in Diäthyläther gelöst, mit wasserfreiem Chlorwasserstoff versetzt und das erhaltene Hydrochlorid-Salz abgetrennt. Man erhält das 1-[N-(1S-Carboäthoxy-3-phenyl-propyl)-S-alanyl]-indolin-2S-carbonsäurehydrochlorid, welches unter Zersetzung bei 140° schmilzt. $[\alpha]_D$ = -77,5° (c = 1,4 in Aethanol).

Beispiel 5: Ein Gemisch von 20,0 g 1-(S-Alanyl)-indolin-2S-carbonsäure, 53,0 g 2-Oxo-4-phenylbutansäure-äthylester, 22 g Diisopropyläthylamin und 130 g 3A-Molekularsieb in 250 ml Aethanol wird bei Zimmertemperatur 30 Minuten gerührt. Eine Lösung von 6,7 g Natriumcyanborhydrid in 75 ml Aethanol wird innerhalb 5 Stunden zugesetzt. Das Reaktionsgemisch wird eine Stunde weiter gerührt, filtriert und unter vermindertem Druck eingedampft. Eine wässerige Suspension des Rückstands wird zweimal mit 150 ml Aether gewaschen. Der pH-Wert wird mit konzentrierter Chlorwasserstoffsäure auf 1,5 eingestellt und es werden 100 ml Methylenchlorid zugegeben. Man rührt eine Stunde und stellt den pH-Wert mit 10%igem wässerigen Natriumhydroxid auf 2,9 ein. Die Methylenchlorid-Schicht wird abgetrennt und die wässerige Phase noch zweimal mit 100 ml Methylenchlorid extrahiert. Die vereinigten Methylenchlorid-Extrakte werden über Magnesiumsulfat getrocknet und unter vermindertem Druck eingedampft. Das erhaltene Produkt wird aus 150 ml Aether umkristallisiert. Man erhält die 1-[N-(1S-Carboäthoxy-3-phenyl-propyl)-S-alanyl]-indolin-2S-carbonsäure, welche unter Zersetzung bei 80° schmilzt. $[\alpha]_D$ = -106° (c = 0,8 in Aethanol).

Beispiel 6: Ein Gemisch von 5,0 g 1-(S-Alanyl)-indolin-2S-carbonsäure, 13,2 g 2-Oxo-4-phenylbuttersäureäthylester und 6 ml Triäthylamin in 115 ml Aethanol wird in Gegenwart von 4,8 g Raney-Nickel und 17 g 3A-Molekularsieb bei 2,7 Atmosphären hydriert. Nach 15 Stunden wird das Reaktionsgemisch filtriert und das Filtrat zur Trockene eingedampft. Der Rückstand wird mit 75 ml Essigsäureäthylester und 50 ml Wasser geschüttelt, wobei man den pH-Wert mit 2-normaler wässeriger Natriumhydroxidlösung auf 8,6 einstellt. Die wässerige Phase wird abgetrennt, mit 20 ml Essigsäureäthylester gewaschen, mit 4-normaler Chlorwasserstoffsäure auf den pH-Wert 4,25 eingestellt und dreimal mit 50 ml Essigsäureäthylester extrahiert. Die vereinigten drei letzten Essigsäureäthylester-Extrakte werden über Natriumsulfat getrocknet und zur Trockene eingedampft. Der Rückstand wird mit wasserfreiem Chlorwasserstoff in Diäthyläther behandelt. Das erhaltene Hydrochloridsalz wird abgetrennt und aus Essigsäureäthylester umkristallisiert. Man erhält das 1-[N-(1R-Carboäthoxy-3-phenyl-propyl)-S-alanyl]-indolin-2S-carbonsäure-hydrochlorid, welches bei 207-208° schmilzt. $[\alpha]_D$ = -109,6° (c = 1,09 in Aethanol).

Beispiel 7: Ein Gemisch von 6,4 g Natrium-3-phenyl-2-oxo-propanoat, 2,0 g 1-(S-Alanyl)-indolin-2S-carbonsäure und 1,6 g Natrium-cyanborhydrid in 50 ml Wasser wird bei Zimmertemperatur mit 1-normaler Chlorwasserstoffsäure auf den pH-Wert 7 eingestellt. Das Reaktionsgemisch wird bei Zimmertemperatur 2 Tage gerührt, durch Zugabe von 10 ml konzentrierter Chlorwasserstoffsäure gelöscht und unter vermindertem Druck eingedampft. Das erhaltene Produkt wird auf einem stark saueren Ionenaustauscher-Säule (Dowex 50, $H^+$-Form) absorbiert und mit 4%igem wässerigem Ammoniak eluiert. Man erhält die 1-[N-(1-Carboxy-2-phenyl-äthyl)-S-alanyl]-indolin-2S-carbonsäure, welche bei 140-143° schmilzt. $[\alpha]_D$ = -92° (c = 1,1 in Aethanol), mit NMR-Peaks bei 1,35, 2,85 und 4,80 ppm.

Beispiel 8: Ein Gemisch von 2,0 g 1-(S-Alanyl)-indolin-2S-carbonsäure, 13 g 3Å-Molekularsieb, 2,4 ml Triäthylamin und 5,0 g Brenztranbensäure-äthylester in 30 ml Aethanol wird 30 Minuten bei Zimmertemperatur gerührt. Eine Lösung von 0,54 g Natrium-cyanborhydrid in 10 ml Aethanol wird innerhalb 2 Stunden zugesetzt. Das Reaktionsgemisch wird über Nacht bei Zimmertemperatur gerührt, mit 4 ml konzentrierter Chlorwasserstoffsäure gelöscht und zur Trockene eingedampft. Das erhaltene Produkt wird auf einem stark saueren Ionenaustauscher-Säule (Dowex 50, $H^+$-Form) absorbiert und die Säule mit 4%igem wässerigem Ammoniak eluiert. Man erhält die 1-[N-(1-Carboäthoxy-äthyl)-S-alanyl]-indolin-2S-carbonsäure, welche in DMSO NMR-Peaks bei 3,8, 1,6 und 1,2 ppm aufweist.

Beispiel 9: Eine Lösung von 0,5 g 1-[N-(1S-Carboäthoxy-3-phenyl-propyl)-S-alanyl]-indolin-2S-carbonsäure in 25 ml Aethanol wird bei 0° mit Ammoniak gesättigt und in einem Druckgefäss 4 Tage stehen gelassen. Die Lösung wird dann eingedampft, der Rückstand in Wasser aufgenommen, das Gemisch mit 3-normaler Chlorwasserstoffsäure bei 0° auf den pH-Wert 3,5 gestellt und mit Methylenchlorid extrahiert. Man erhält die 1-[N-(1S-Carbamoyl-3-phenyl-propyl)-S-alanyl]-indolin-2S-carbonsäure, welche im IR-Spektrum Peaks bei 1710, 1660 und 3220 $cm^{-1}$ aufweist.

Beispiel 10: Herstellung von 10'000 Tabletten mit einem Gehalt von je 5 mg der aktiven Substanz:

Bestandteile:

| | |
|---|---|
| 1-[N-(1-Carboxy-3-phenylpropyl)-S-alanyl]-indolin-2S-carbonsäure | 50 g |
| Milchzucker | 1157 g |
| Maisstärke | 75 g |
| Polyäthylenglykol 6000 | 75 g |
| Talkpulver | 5 g |
| Magnesiumstearat | 18 g |
| Gereinigtes Wasser | q.s. |

Verfahren: Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten mit 6,4 mm Durchmesser, welche eine Bruchrille aufweisen, gepresst.

Beispiel 11: Herstellung von 10'000 Kapseln mit einem Gehalt von je 10 mg der aktiven Substanz:

Bestandteile:

| | |
|---|---|
| 1-[N-(1S-Carboäthoxy-3-phenylpropyl)-S-alanyl]-indolin-2S-carbonsäure | 100 g |
| Milchzucker | 1800 g |
| Talkpulver | 100 g |

Verfahren: Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 3 werden mit je 200 mg des Gemisches mit einer Füllmaschine gefüllt.

In analoger Weise werden Tabletten oder Kapseln von anderen erfindungsgemässen Verbindungen, z.B. solchen, die in den weiteren Beispielen hier illustriert sind, hergestellt.

Beispiel 12: Ein Gemisch von 1-(S-Alanyl)-indolin-2S-carbonsäure (1oo g), 2000 ml wasserfreies Aethanol, 29,9 g Essigsäure und 270 g 4-Phenyl-2-oxo-butansäure-äthylester wird 2 Stunden bei Zimmertemperatur gerührt. Dann gibt man innerhalb 5 Stunden 33,3 g Natrium-cyanborhydrid in 300 ml wasserfreiem Aethanol, gleichmässig dazu, wobei man die Temperatur zwischen 20-24° hält. Das Reaktionsgemisch wird über Nacht bei Zimmertemperatur gerührt, dann auf 0° gekühlt, mit 100 ml 12-molarer Chlorwasserstoffsäure auf einmal versetzt und die Kühlung unterbrochen. Das Gemisch wird 1 Stunde gerührt und dann bei 3 mm Hg und 20-24° eingedampft. Der erhaltene Sirup wird zu einem stark gerührten Gemisch von 600 g Eis, 600 ml 6-normalem Natriumhydroxid, und 1000 ml Aether gegeben. Die wässerige Phase wird abgetrennt und mit 1000 ml frischem Aether gewaschen. Die wässerige Lösung (pH-Wert ca. 1,24) wird mit 1000 ml Methylenchlorid versetzt, auf 0-5° gekühlt und mit ca. 93 ml 12-normaler Chlorwasserstoffsäure auf den pH-Wert 4,3 schnell angesäuert. Das Methylenchlorid wird abgetrennt und das wässerige Gemisch zweimal mit je 500 ml Methylenchlorid extrahiert. Die vereinigten organischen Lösungen werden über wasserfreiem Natriumsulfat getrocknet, durch Filtrieren geklärt und bei 20-24° und 3 mm Hg eingedampft. Man erhält die 1-[N-(1S-Carboäthoxy-3-phenylpropyl)-S-alanyl]-indolin-2S-carbonsäure als einen Schaum.

Man löst 194 g der erhaltenen rohen Base in 500 ml Essigsäureäthylester und gibt die Lösung tropfenweise zu einer stark gerührten Lösung von 42 g Phosphorsäure in 2300 ml Aethanol. Das Gemisch wird über Nacht bei 5° gehalten, dann eine Stunde gerührt und der Niederschlag abfiltriert. Der Filterkuchen wird 3-mal mit je 100 ml Essigsäureäthylester und 3 mal mit je 100 ml Aether gewaschen und bei 24° und 3 mm Hg getrocknet. Das erhaltene rohe Phosphatsalz wird aus einem Gemisch von absolutem Aethanol/Essigsäureäthylester umkristallisiert und bei 24° und 0,01 mm Hg getrocknet. Man erhält das 1-[N-(1S-Carboäthoxy-3-phenylpropyl)-S-alanyl]-indolin-2S-carbonsäure-1:1-phosphat, welches bei 125-129° schmilzt.
$[\alpha]_D^{23}$ = -87,2° (c = 1 in Methanol).

Beispiel 13: Eine Lösung von 3,67 g Natriumhydroxid in 100 ml Aethanol wird bei Zimmertemperatur mit einer Suspension von 16,0 g 1-[N-(1S-Carboäthoxy-3-phenylpropyl)-S-alanyl]-indolin-2S-carbonsäure-phosphorsäuresalz in 100 ml Aethanol versetzt. Das Gemisch wird 30 Minuten gerührt und dann filtriert. Das Filtrat wird eingedampft, der Rückstand in 100 ml Diäthyläther gelöst, filtriert und über Nacht bei Zimmertemperatur stehen gelassen. Das kristalline Produkt wird abfiltriert, mit Diäthyläther gewaschen und im Vakuum getrocknet. Man erhält das Natriumsalz der 1-[N-(1S-Carboäthoxy-3-phenylpropyl)-S-alanyl]-indolin-2S-carbonsäure. F. 190-192°.
$[\alpha]_D$ = -110° (c = 0,2 in Aethanol).

Beispiel 14: Man gibt 1,6 g Natriumhydroxid in 50 ml Wasser zu 8,0 g 1-[N-(1S-Carboäthoxy-3-phenylpropyl)-S-alanyl]-indolin-2S-carbonsäure in 50 ml Methanol. Das Reaktionsgemisch wird 3 Stunden bei Zimmertemperatur gerührt und dann eingedampft. Der Rückstand wird in 100 ml Wasser aufgenommen und zweimal mit je 50 ml Diäthyläther extrahiert. Die wässerige Schicht wird mit konz. Chlorwasserstoffsäure auf den pH-Wert 3 eingestellt. Der Niederschlag wird abfiltriert und aus Essigsäureäthylester umkristallisiert. Man erhält die 1-[N-(1S-Carboxy-3-phenylpropyl)-S-alanyl]-indolin-2S-carbonsäure, welche bei 159-160° schmilzt.
$[\alpha]_D$ = -70,4 (c = 0,6 in Aethanol).

Beispiel 15: Eine Lösung von 3,0 g 1-[N-(1S-Carboäthoxy-3-phenylpropyl)-S-alanyl]-indolin-2S-carbonsäure-natriumsalz in 30 ml Methanol, welches 100 mg Natriumcarbonat enthält, wird 10 Stunden unter Rückfluss gekocht und dann eingedampft. Der Rückstand wird in 150 ml

Diäthyläther aufgenommen und filtriert. Das Filtrat wird auf ein Volumen von 50 ml eingedampft und mit 50 ml Hexan versetzt. Der erhaltene Niederschlag wird abfiltriert. Man erhält das 1-[N-(1S-Carbomethoxy-3-phenylpropyl)-S-alanyl]-indolin-2S-carbonsäure-natriumsalz, welches bei 140-145° schmilzt.
$[\alpha]_D$ =-123,7° (c = 0,8 in Methanol).

Ausgangsstoff für das Beispiel 3/3

Eine Lösung von 5,0 g Indolin-2S-carbonsäure-äthylester-hydrochlorid, 5,5 g N-Carbobenzyloxy-L-valin und 3,0 ml Triäthylamin in 200 ml Methylenchlorid wird mit 4,4 g 1-(3-Dimethylaminopropyl)-3-äthyl-carbodiimid-hydrochlorid versetzt. Das Reaktionsgemisch wird über Nacht bei Zimmertemperatur gerührt und dann mit 75 ml Wasser, 75 ml 2-normaler wässeriger Chlorwasserstoffsäure und 75 ml gesättigter wässeriger Natriumhydrogencarbonatlösung gewaschen. Die organische Schicht wird über Magnesiumsulfat getrocknet und eingedampft. Man erhält den 1-(N-Carbobenzyloxy-L-valyl)-indolin-2S-carbonsäure-äthylester.

Man versetzt 9,3 g 1-(N-Carbobenzyloxy-L-valyl)-indolin-2S-carbonsäure-äthylester in 75 ml Methanol mit 1,05 g Natriumhydroxid in 75 ml Wasser. Das Reaktionsgemisch wird bei Zimmertemperatur 3 Stunden gerührt und dann eingedampft. Der Rückstand wird in 200 ml Wasser aufgenommen und mit 75 ml Methylenchlorid gewaschen. Die wässerige Schicht wird mit 5 ml konz. Chlorwasserstoffsäure angesäuert und dann dreimal mit je 75 ml Methylenchlorid extrahiert. Die vereinigten organischen Extrakte werden über Natriumsulfat getrocknet und eingedampft. Man erhält die 1-(N-Carbobenzyloxy-L-valyl)-indolin-2S-carbonsäure.

Zu 8,7 g 1-(N-Carbobenzyloxy-L-valyl)-indolin-2S-carbonsäure in 100 ml 85%igem wässerigem Aethanol gibt man 0,5 g 10%igen Palladiumauf Kohle Katalysator. Das Reaktionsgemisch wird bei Zimmertemperatur

und atmosphärischem Druck 3 Stunden hydriert und eingedampft. Man erhält die 1-(L-Valyl)-indolin-2S-carbonsäure, welche bei 194-196° schmilzt.
$[\alpha]_D$ = -63,2° (c = 0,9 in 50%igem wässerigem Aethanol).

Ausgangsstoff für das Beispiel 3/4

Zu 10,0 g Indolin-2S-carbonsäureäthylester, 10,7 g α-N-t-Butoxycarbonyl-ε-N-benzyloxycarbonyl-L-lysin und 6,1 ml Triäthylamin in 250 ml Methylenchlorid gibt man 8,8 g 1-(3-Dimethylaminopropyl)-3-äthylcarbodiimid-hydrochlorid. Das Reaktionsgemisch wird über Nacht bei Zimmertemperatur gerührt, mit 75 ml Wasser, 75 ml 2-normaler wässeriger Chlorwasserstoffsäure und 75 ml gesättigter wässeriger Natriumbicarbonatlösung gewaschen. Die organische Schicht wird über Magnesiumsulfat getrocknet und eingedampft. Man erhält den 1-(α-N-t-Butoxycarbonyl-ε-N-benzyloxycarbonyl-L-lysyl)-indolin-2S-carbonsäureäthylester.

Eine Lösung von 22,5 g 1-(α-N-t-Butoxycarbonyl-ε-N-benzyloxycarbonyl-L-lysyl)-indolin-2S-carbonsäure-äthylester in 150 ml Methanol wird mit einer Lösung von 1,8 g Natriumhydroxid in 150 ml Wasser versetzt. Das Reaktionsgemisch wird bei Zimmertemperatur 3 Stunden gerührt und dann eingedampft. Der Rückstand wird in 300 ml Wasser aufgenommen und mit 100 ml Methylenchlorid gewaschen. Die wässerige Schicht wird mit 5 ml konz. Chlorwasserstoffsäure angesäuert und dreimal mit je 100 ml Methylenchlorid extrahiert. Die kombinierten organischen Extrakte werden über Magnesiumsulfat getrocknet und eingedampft. Man erhält die 1-(α-N-t-Butoxycarbonyl-ε-N-benzyloxycarbonyl-L-lysyl)-indolin-2S-carbonsäure.

Zu 7,6 g 1-(α-N-t-Butoxycarbonyl-ε-N-benzyloxycarbonyl-L-lysyl)-indolin-2S-carbonsäure in 13 ml Eisessig, 20 ml Methylenchlorid und 13 ml Anisol gibt man bei 0° 40 ml Trifluoressigsäure. Das Reaktionsgemisch lässt man unter 30-minutigem Rühren sich auf Zimmertemperatur erwärmen und dann dampft es ein. Der Rückstand wird aus Diäthyläther umkristallisiert. Man erhält das Trifluoressigsäure-salz der 1-(ε-N-Benzyloxycarbonyl-L-lysyl)-indolin-2S-carbonsäure.

Kardiovaskulare Pharmakologie von Verbindungen der Erfindung

Die Prüfung der Wirkung von Verbindungen ist gemäss den Methoden für die Auswertung der Hemmung des Enzyms, welches das Angiotensin umwandelt [angiotensin converting enzyme = ACE] durchgeführt worden. In der biochemischen Beurteilung der in vitro ACE-Hemmung (ACE inhibition = ACEI] wird die Aktivität einer Verbindung im Kaninchen-Lungengewebe bestimmt.

In der in vivo Prüfungsmethode wird zuerst durch Verabreichung des Angiotensins I (AI) an das Versuchstier eine Blutdrucksteigerung hervorgerufen. Es wird dann die Hemmung der einzelnen Verbindungen auf diese Blutdrucksteigerung gemessen.

I. Biochemische Testmethode

Es wurde ein Kaninchen-Lungengewebe-Präparat [(Das and Saffer, J. Biol. Chem. 250: 6762 (1975)] für die Bestimmung von ACE nach der Methode von Cheung und Cushman [Cheung and Cushman, Biochim. Biophys. Acta 293, 451 (1973)] verwendet. In dieser Methode wird die Menge des Histidyl-leucins, welche aus einem synthetischen Substrat innerhalb einer Inkubation von 30 Minuten bei 37° freigesetzt wird, spektrophotometrisch bestimmt. Die $IC_{50}$-Werte der ACE-Hemmung werden dann graphisch berechnet. Der $IC_{50}$-Wert ist die Konzentration (in Mol) der getesteten Verbindung, welche die in Abwesenheit der Testsubstanz entstehende Menge des Histidyl-leucins um 50% herabsetzt.

II. Methode der Hemmung der durch Angiotensin I (AI) hervorgerufenen Blutdrucksteigerung, durch intravenöse Verabreichung der Testverbindung (% AI-Hemmung)

In diesen Untersuchungen wird wie oben beschrieben, je ein Katheter in eine femurale Arterie und in eine Wadenvene der Ratte eingeführt.

Der arterielle Druck wurde von dem Arterienkatheter aus registriert, während man Angiotensin I (AI) und die einzelnen Testverbindungen durch den Venenkatheter injiziert hatte. Die Hemmung der durch Angiotensin I verursachten Blutdrucksteigerung ist in der Tabelle als Durchschnittswert 30 Minuten nach der Verabreichung von ausgewählten Dosen der jeweiligen Verbindung wiedergegeben. Dabei werden solche Dosierungen ausgewählt, so dass u.a. der $ID_{50}$ (i.v.)-Wert ermittelt werden kann.

Ergebnisse:

| Verbindung des Beispiels | In vitro ACEI $IC_{50}$ (M) | Hemmung der durch Angiotensin I (AI) verursachten Blutdrucksteigerung | |
|---|---|---|---|
| | | i.v. Dosis (mg/kg) | % AI Hemmungen |
| 1 | $6 \times 10^{-9}$ | 0.1 | 80 |
| 6 | $8 \times 10^{-6}$ | 0.1 | 35 |
| 7 | $3 \times 10^{-8}$ | 1.0 | 80 |
| 4 | $3 \times 10^{7}$ | 0.1 | 95 |
| | | 0.06 | 80 |
| | | 0.03 | 33 |

Patentansprüche

(für alle benannten Länder ausser Oesterreich)

1. Verbindungen der allgemeinen Formel I

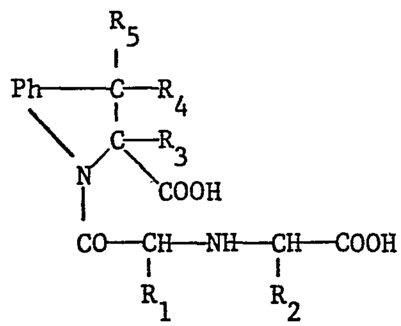

(I),

worin Ph unsubstituiertes 1,2-Phenylen oder durch einen bis drei, gleiche oder verschiedene Substituenten der Gruppe Niederalkyl, Niederalkoxy, Niederalkylendioxy, Hydroxy, Halogen oder Trifluormethyl substituiertes 1,2-Phenylen bedeutet, $R_1$ für Wasserstoff, Niederalkyl oder Amino-niederalkyl steht, $R_2$ Niederalkyl oder HPh-Niederalkyl bedeutet, und jedes der Symbole $R_3$, $R_4$ und $R_5$ für Wasserstoff oder Niederalkyl steht; ihre Amide, Mono- oder Di-niederalkylamide, Niederalkylester, (Amino, Mono- oder Di-niederalkylamino, Carboxy oder Carboniederalkoxy)-niederalkylester, und ihre Salze.

2. Verbindungen der im Anspruch 1 gezeigten Formel I, worin Ph unsubstituiertes 1,2-Phenylen oder durch einen oder zwei, gleiche oder verschiedene Substituenten der Gruppe Niederalkyl, Niederalkoxy, Hydroxy und Halogen, oder durch eine Alkylendioxy- oder Trifluormethylgruppe substituiertes 1,2-Phenylen bedeutet, $R_1$ für Wasserstoff, Niederalkyl oder ω-Amino-niederalkyl steht, $R_2$ Niederalkyl oder ω-HPh-Niederalkyl bedeutet, und jedes der Symbole $R_3$, $R_4$ und $R_5$ für Wasserstoff oder Niederalkyl steht, ihre Amide, Mono- oder Di-niederalkylamide, Niederalkylester, (Amino, Mono- oder Di-niederalkylamino, Carboxy oder Carbo-niederalkoxy)-niederalkylester und Salze.

3. Verbindungen der im Anspruch 1 gezeigten Formel I, worin Ph ein gegebenenfalls durch Niederalkyl, Niederalkoxy, Niederalkylendioxy, Hydroxy, Halogen oder Trifluormethyl monosubstituiertes 1,2-Phenylen bedeutet, $R_1$ für Niederalkyl oder ω-Amino-niederalkyl steht, $R_2$ Niederalkyl oder ω-HPh-Niederalkyl bedeutet, und jedes der Symbole $R_3$, $R_4$ und $R_5$ Wasserstoff oder Methyl bedeutet, ihre Amide, Mono- oder Di-niederalkylamide, Niederalkylester, (Amino, Mono- oder Di-niederalkylamino, Carboxy oder Carbo-niederalkoxy)-niederalklester und Salze.

4. Verbindungen der allgemeinen Formel II

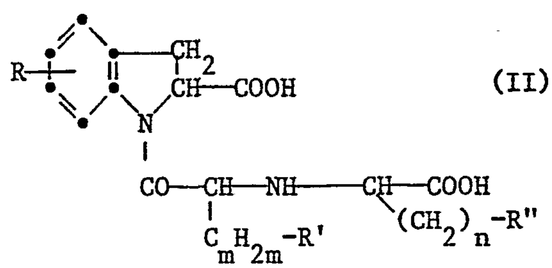

(II) ,

worin R Wasserstoff, Alkyl oder Alkoxy mit höchstens je 4 Kohlenstoffatomen, Hydroxy, Halogen oder Trifluormethyl bedeutet, m für eine ganze Zahl von 1 bis 4 steht, n eine ganze Zahl von 1 bis 3 bedeutet, R' für Wasserstoff oder Amino steht, und R" Wasserstoff oder R-Phenyl bedeutet; ihre Mono- oder Bisamide, die Mono- oder Bis-(niederalkyl oder ω-amino-niederalkyl)-ester, und Salze.

5. Verbindungen der im Anspruch 4 gezeigten Formel II in der Form von ihren Indolin-2S-carboxy chiralen Epimeren.

6. 1-[N-(1-Carboxy-3-phenylpropyl)-S-alanyl]-indolin-2S-carbonsäure und ihre Salze.

7. 1-[N-(1S-Carboäthoxy-3-phenylpropyl)-S-alanyl]-indolin-2S-carbonsäure und ihre Salze.

8. 1-[N-(1R-Carboäthoxy-3-phenylpropyl)-S-alanyl]-indolin-2S-carbonsäure und ihre Salze.

9. 1-[N-(1-Carboxy-2-phenyläthyl)-S-alanyl]-indolin-2S-carbonsäure und ihre Salze.

10. Pharmazeutische Präparate enthaltend Verbindungen der im Anspruch 1 gezeigten Formel I, ihre Amide, Mono- und Di-niederalkylamide, Niederalkylester, (Amino, Mono- oder Di-niederalkylamino, Carboxy oder Carboniederalkoxy)-niederalkylester, und ihre Salze, zusammen mit einem pharmazeutischen Trägermaterial.

11. Die im Anspruch 1 genannten Verbindungen zur Anwendung in einem Verfahren zur therapeutischen Behandlung des menschlichen oder tierischen Körpers.

12. Die im Anspruch 1 genannten Verbindungen als hypotensive, antihypertensive und bradikardische Mittel.

13. Verwendung der im Anspruch 1 genannten Verbindungen zur Herstellung von pharmazeutischen Präparaten.

14. Verfahren zur Herstellung von den im Anspruch 1 genannten Verbindungen der Formel I und ihren im Anspruch 1 genannten Derivaten, dadurch gekennzeichnet, dass man

1) eine Schiff'sche Base der allgemeinen Formel III

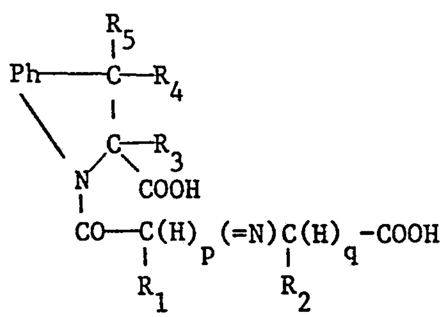

(III)

oder ihre genannten funktionellen Derivate, worin eines der Symbole p und q für Null steht und das andere 1 bedeutet, hydriert, oder

2) eine Verbindung der allgemeinen Formel IV

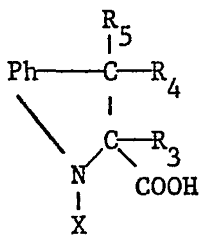

(IV)

oder ihre genannten funktionellen Derivate, mit einem reaktionsfähigen funktionellen Derivat einer Verbindung der allgemeinen Formel V

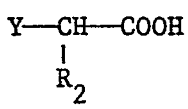

(V) ,

in welcher X Wasserstoff bedeutet und Y für die Gruppierung $HOCO-CHR_1-N-COOR_o$ steht, worin $R_o$ t-Butyl oder Benzyl bedeutet; oder X für $CO-CHR_1-Z$ steht und eines der Symbole Y und Z Amino bedeutet und das andere für eine reaktionsfähige veresterte Hydroxygruppe steht, kondensiert, und die genannte $COOR_o$-Gruppe in Wasserstoff umwandelt, oder

3) eine Verbindung der allgemeinen Formel VI

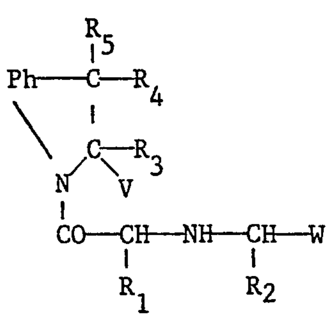

(VI) ,

worin mindestens eines der Symbole V und W Cyan bedeutet, und das andere für die genannte freie, amidierte oder veresterte Carboxygruppe steht, hydrolysiert oder alkoholisiert, oder

4) in einer Verbindung der allgemeinen Formel VII

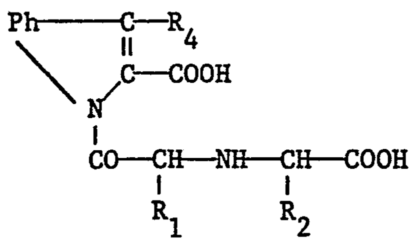

oder in ihren genannten funktionellen Derivaten, den Indolteil zum Indolinteil hydriert, und, wenn erwünscht, eine erhaltene Verbindung in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

15. Die nach dem Verfahren des Anspruchs 14 erhältlichen Verbindungen.

Patentansprüche

(für Oesterreich)

1. Verfahren zur Herstellung von neuen 1-Carboxy-(azaalkanoyl oder azaaralkanoyl)-indolin-2-carbonsäuren der allgemeinen Formel I

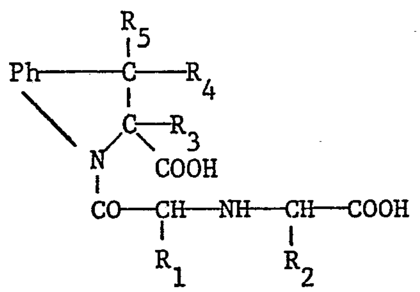

(I) ,

worin Ph unsubstituiertes 1,2-Phenylen oder durch einen bis drei, gleiche oder verschiedene Substituenten der Gruppe Niederalkyl, Niederalkoxy, Niederalkylendioxy, Hydroxy, Halogen oder Trifluormethyl substituiertes 1,2-Phenylen bedeutet, $R_1$ für Wasserstoff, Niederalkyl oder Amino-niederalkyl steht, $R_2$ Niederalkyl oder HPh-Niederalkyl bedeutet, und jedes der Symbole $R_3$, $R_4$ und $R_5$ für Wasserstoff oder Niederalkyl steht; ihren Amiden, Mono- oder Di-niederalkylamiden, Niederalkylestern, (Amino, Mono- oder Di-niederalkylamino, Carboxy oder Carboniederalkoxy)-niederalkylestern, und ihren Salzen, dadurch gekennzeichnet, dass man

1) eine Schiff'sche Base der allgemeinen Formel III

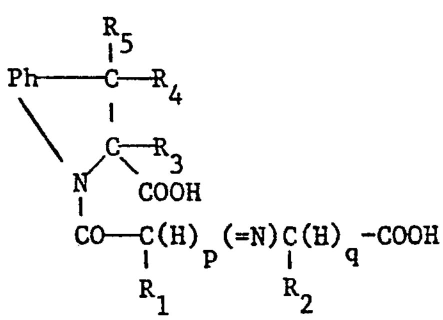

(III)

oder ihre genannten funktionellen Derivate, worin eines der Symbole p und q für Null steht und das andere 1 bedeutet, hydriert, oder

2) eine Verbindung der allgemeinen Formel IV

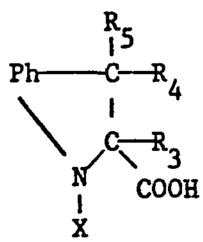

(IV)

oder ihre genannten funktionellen Derivate, mit einem reaktionsfähigen funktionellen Derivat einer Verbindung der allgemeinen Formel V

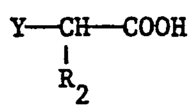

(V) ,

in welcher X Wasserstoff bedeutet und Y für die Gruppierung $HOCO-CHR_1-N-COOR_o$ steht, worin $R_o$ t-Butyl oder Benzyl bedeutet; oder X für $CO-CHR_1-Z$ steht und eines der Symbole Y und Z Amino bedeutet und das andere für eine reaktionsfähige veresterte Hydroxygruppe steht, kondensiert, und die genannte $COOR_o$-Gruppe in Wasserstoff umwandelt, oder

3) eine Verbindung der allgemeinen Formel VI

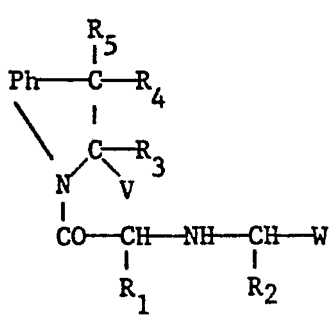

(VI) ,

worin mindestens eines der Symbole V und W Cyan bedeutet, und das andere für die genannte freie, amidierte oder veresterte Carboxygruppe steht, hydrolysiert oder alkoholisiert, oder

4) in einer Verbindung der allgemeinen Formel VII

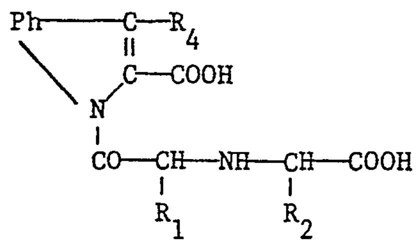

oder in ihren genannten funktionellen Derivaten, den Indolteil zum Indolinteil hydriert, und, wenn erwünscht, eine erhaltene Verbindung in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Razemate in die optischen Antipoden aufspaltet.

2. Verfahren nach Anspruch 1, dadurch gekennzeichnet, dass man Verbindungen der im Anspruch 1 gezeigten Formel I, worin Ph unsubstituiertes 1,2-Phenylen oder durch einen oder zwei, gleiche oder verschiedene Substituenten der Gruppe Niederalkyl, Niederalkoxy, Hydroxy und Halogen, oder durch eine Alkylendioxy- oder Trifluormethylgruppe substituiertes 1,2-Phenylen bedeutet, $R_1$ für Wasserstoff, Niederalkyl oder ω-Amino-niederalkyl steht, $R_2$ Niederalkyl oder ω-HPh-Niederalkyl bedeutet, und jedes der Symbole $R_3$, $R_4$ und $R_5$ für Wasserstoff oder Niederalkyl steht, ihre Amide, Mono- oder Di-niederalkylamide, Niederalkylester, (Amino, Mono- oder Di-niederalkylamino, Carboxy oder Carbo-niederalkoxy)-niederalkylester und Salze, herstellt.

3. Verfahren nach Anspruch 1, dadurch gekennzeichnet, dass man Verbindungen der im Anspruch 1 gezeigten Formel I, worin Ph ein gegebenenfalls durch Niederalkyl, Niederalkoxy, Niederalkylendioxy, Hydroxy, Halogen oder Trifluormethyl monosubstituiertes 1,2-Phenylen

bedeutet, $R_1$ für Niederalkyl oder ω-Amino-niederalkyl steht, $R_2$ Niederalkyl oder ω-HPh-Niederalkyl bedeutet, und jedes der Symbole $R_3$, $R_4$ und $R_5$ Wasserstoff oder Methyl bedeutet, ihre Amide, Mono- oder Di-niederalkylamide, Niederalkylester, (Amino, Mono- oder Di-niederalkylamino, Carboxy oder Carbo-niederalkoxy)-niederalkylester und Salze, herstellt.

4. Verfahren nach Anspruch 1, dadurch gekennzeichnet, dass man Verbindungen der allgemeinen Formel II

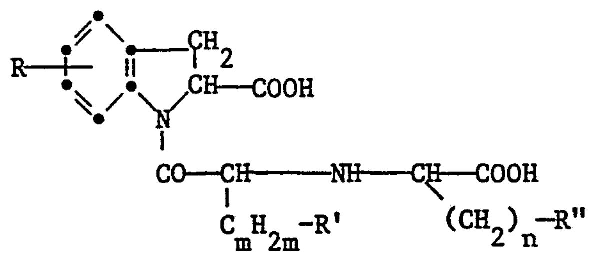

worin R Wasserstoff, Alkyl oder Alkoxy mit höchstens je 4 Kohlenstoffatomen, Hydroxy, Halogen oder Trifluormethyl bedeutet, m für eine ganze Zahl von 1 bis 4 steht, n eine ganze Zahl von 1 bis 3 bedeutet, R' für Wasserstoff oder Amino steht, und R" Wasserstoff oder R-Phenyl bedeutet; ihre Mono- oder Bisamide, die Mono- oder Bis-(niederalkyl oder ω-amino-niederalkyl)-ester, und Salze, herstellt.

5. Verfahren nach Anspruch 1, dadurch gekennzeichnet, dass man Verbindungen der im Anspruch 4 gezeigten Formel II in der Form von ihren Indolin-2S-carboxy chiralen Epimeren herstellt.

6. Verfahren nach Anspruch 1, dadurch gekennzeichnet, dass man 1-[N-(1-Carboxy-3-phenylpropyl)-S-alanyl]-indolin-2S-carbonsäure und ihre Salze herstellt.

7. Verfahren nach Anspruch 1, dadurch gekennzeichnet, dass man 1-[N-(1S-Carboäthoxy-3-phenylpropyl)-S-alanyl]-indolin-2S-carbonsäure und ihre Salze herstellt.

8. Verfahren nach Anspruch 1, dadurch gekennzeichnet, dass man 1-[N-(1R-Carboäthoxy-3-phenylpropyl)-S-alanyl]-indolin-2S-carbonsäure und ihre Salze herstellt.

9. Verfahren nach Anspruch 1, dadurch gekennzeichnet, dass man 1-[N-(1-Carboxy-2-phenyläthyl)-S-alanyl]-indolin-2S-carbonsäure und ihre Salze herstellt.

10. Verfahren zur Herstellung eines pharmazeutischen Präparates, gekennzeichnet durch die Verarbeitung eines erfindungsgemässen Wirkstoffes nach einem der Ansprüche 1 bis 7, mit einem pharmazeutischen Trägermaterial.

11. Verfahren zur Herstellung eines pharmazeutischen Präparates, gekennzeichnet durch die Verarbeitung eines erfindungsgemässen Wirkstoffes nach einem der Ansprüche 8 und 9, mit einem pharmazeutischen Trägermaterial.

0051301

Europäisches Patentamt

# EUROPÄISCHER RECHERCHENBERICHT

Nummer der Anmeldung

EP 81 10 9378

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P/X | EP - A - 0 037 231 (WARNER-LAMBERT CO.)<br>* Seite 16, Tabelle * | 1,2,6-9 |
| P/A | EP - A - 0 024 852 (AM. HOME PRODUCTS)<br>* ganzes Dokument * | 1 |
| D | EP - A - 0 012 401 (MERCK & CO)<br>* ganzes Dokument * | 1 |

KLASSIFIKATION DER ANMELDUNG (Int Cl.³)

C 07 C 103/52
C 07 D 209/42
A 61 K 31/02

RECHERCHIERTE SACHGEBIETE (Int. Cl.³)

C 07 C 103/52
C 07 D 209/42
A 61 K 31/02

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 05.02.1982 | GRAMAGLIA |

EPA form 1503.1 06.78